# EUROPÄISCHE PATENTSCHRIFT

(11) **EP 0 005 249 B1**
(45) Veröffentlichungstag und Bekanntmachung des Hinweises auf die Patenterteilung: **02.09.1981**
(21) Anmeldenummer: 79101287.5
(22) Anmeldetag: 30.04.1979
(51) Int. Cl.: C07D 231/12, C07D 231/14, C07D 249/08, C07D 249/10, C07D 249/04, C07D 233/56, C07D 233/64, C07D 233/66, A01N 43/50, A01N 43/56, A01N 43/64

(54) **Substituierte Alkanyl-azolyl-oximcarbamate, Verfahren zu ihrer Herstellung und ihre Verwendung als Insektizide, Akarizide und Nematozide**
Substituted alkanyl-azolyl carbamates, process for their preparation and their use as insecticides, acaricides and nematocides
Carbamates substitués d'alkanyl-azolyl-oximes, procédé pour leur préparation et leur utilisation comme insecticides, acaricides et nématocides

(30) Priorität: 10.05.1978 DE 2820361
(43) Veröffentlichungstag der Anmeldung: 14.11.1979
(73) Patentinhaber: BAYER AG, 51368 Leverkusen (DE)
(72) Erfinder: Stetter, Jörg, Dr., D-5600 Wuppertal 1 (DE); Homeyer, Bernhard, Dr., D-5090 Leverkusen 3 (DE); Hammann, Ingeborg, Dr., D-5000 Koeln 1 (DE)

## Beschreibung

Die vorliegende Erfindung betrifft neue substituierte Alkanyl-azolyl-oximcarbamate, mehrere Verfahren zu ihrer Herstellung und ihre Verwendung als Insektizide, Akarizide und Nematozide.

Es ist bereits bekannt geworden, daß im Alkanylrest gegebenenfalls substituierte Alkanyl-azolyl-oximcarbamate gute insektizide, akarizide und nematozide Eigenschaften aufweisen (vergleiche DE-A-2 613 167 und DE-A-2 635 883).

Die Wirkung der bisher synthesierten Verbindungen ist jedoch, insbesondere bei niedrigen Aufwandmengen, nicht immer ganz befriedigend.

Es wurden neue im Alkanylrest substituierte Alkanyl-azolyl-oximcarbamate der allgemeinen Formel (I) 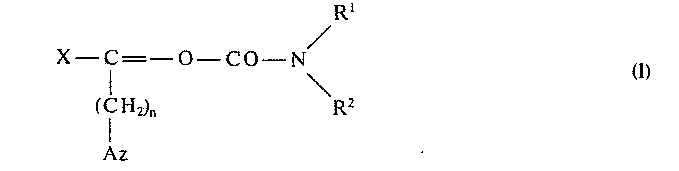 in welcher
Az für gegebenenfalls durch Chlor, Methyl, Äthyl, Nitro oder Methylmercapto substituiertes Pyrazol-1-yl Imidazol-1-yl, 1,2,4-Triazol-1-yl, 1,2,3-Triazol-1-yl und 1,3,4-Triazol-1-yl steht,
R¹ für Wasserstoff oder Alkyl steht,
R² für Wasserstoff, Alkyl, Allyl, Halogenalkyl oder Alkoxyalkyl, sowie die Gruppe -(S)m-R³ steht, falls R' für Alkyl steht,
R³ für Alkyl, Halogenalkyl, gegebenenfalls durch Chlor oder Trifluormethyl substituiertes Phenyl, Alkoxycarbonyl, die Gruppe - NR4R5 sowie für den gleichen Rest steht, an den die Gruppe - (S)m - R³ gebunden ist,
R⁴ für Alkyl steht,
R⁵ für Alkyl, Dialkylcarbamoyl, Alkoxycarbonyl oder gegebenenfalls durch Halogen, Alkyl mit 1 -2 Kohlenstoffatomen oder Halogenalkyl mit 1 -2 Kohlenstoffatomen und bis zu 5 Halogenatomen substituiertes Phenylsulfonyl steht oder
R⁴ und R5 gemeinsam mit dem N-Atom für Piperidino oder Morpholino steht,
X für C₁-₄-Alkyl steht, das ein- oder zweifach substituiert ist durch Halogen, Acyloxy, Carbamoyl-, Alkylcarbamoyl- und Dialkylcarbamoyloxy mit jeweils 1 bis 4 Kohlenstoffatomen in jedem Alkylteil; Alkylsulfonyloxy mit 1 bis 4 Kohlenstoffatomen, Hydroxy, Nitro, Cyano, Alkoxycarbonyl mit 1 bis 4 Kohlenstoffatomen im Alkylteil, sowie Alkoxy und Alkylthio mit jeweils 1 bis 4 Kohlenstoffatomen, wobei die Alkylgruppen von 2 Alkoxy- bzw. Alkylthio-Substituenten auch in Form eines 5- oder 6gliedrigen Ringes miteinander verbunden sein können,
m für 1 oder 2 steht und
n für 0 oder 1 steht

und deren physiologisch verträgliche Säureadditions-Salze und Metallsalz-Komplexe gefunden. Sie weisen starke insektizide, akarizide und nematozide Eigenschaften auf.

Die Verbindungen der Formel (I) können in der syn- oder anti-Form vorliegen; vorwiegend fallen sie als Gemische beider Formen an.

Man erhält die substituierten Alkanyl-azolyl-oximcarbamate der Formel (I), wenn man
a) Oxime der Formel (II) 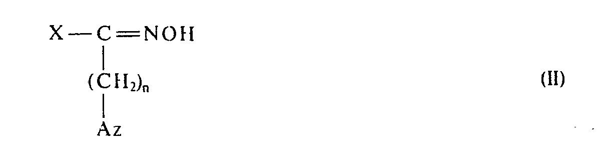 in welcher
   Az, X und n die oben angegebene Bedeutung haben, mit Carbamoylhalogeniden der Formel (III) 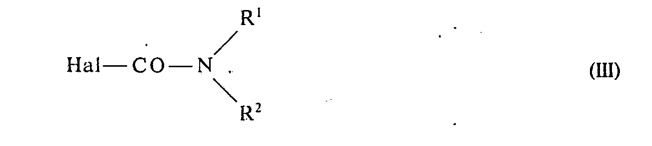 in welcher
   R¹ und R² die oben angegebene Bedeutung haben und
   Hal für Fluor oder Chlor steht,

   entweder in Gegenwart eines Verdünnungsmittels und eines säurebindenden Mittels oder in Gegenwart eines Verdünnungsmittels und Natriumhydrid umsetzt, oder
b) Oxime der Formel (H) mit Isocyanaten der Formel (IV) 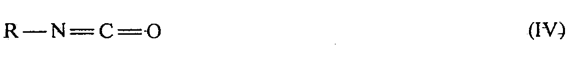 in welcher
   R für Alkyl, Allyl oder Alkoxyalkyl steht,
   in Gegenwart eines Verdünnungsmittels und gegebenenfalls in Gegenwart eines Katalysators umsetzt, oder
c) Oxime der Formel (II) mit Phosgen und anschließend mit Aminen der Formel V 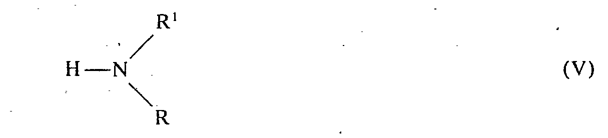 in welcher
   R und R¹ die oben angegebene Bedeutung haben und
   R zusätzlich für Wasserstoff steht,

   entweder in Gegenwart eines Verdünnungsmittels und eines säurebindenden Mittels oder in Gegenwart eines Verdünnungsmittels und Natriumhydrid umsetzt, oder
d) gegebenenfalls die nach den Verfahren (a), (b) und (c) erhältlichen Oximcarbamate der Formel (VI) 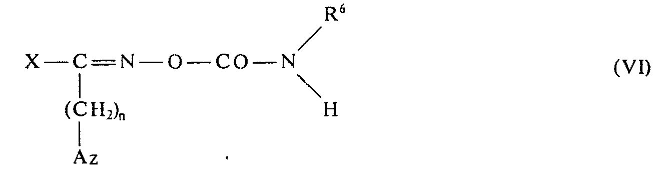 in weicher
   Az, X und n die oben angegebene Bedeutung haben und
   R⁶ fürAlkyl mit 1 bis 4 Kohienstoffatomen steht,
   mit Sulfenchloriden der Formel (VII) 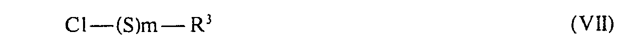 in welcher
   R³ und m die oben angegebene Bedeutung haben,
   in Gegenwart eines Verdünnungsmittels und eines säurebindenden Mittels umsetzt.

Weiterhin können die erfindungsgemäß erhältlichen substituierten AJkanyl-azolyl-oximcarbamate der Formel (1) durch Umsetzung mit Säuren in die Salze überführt werden, bzw. durch Reaktion mit Metallsalzen die entsprechenden Metallkomplexe erhalten werden.

Überraschenderweise zeigen die erfindungsgemäßen substituierten Alkanyl-azolyi-oximcarbamate eine höhere insektizide, akarizide und nematozide Wirkung als die bekannten unsubstituierten Alkanyl-azolyl-oximcarbamate, welche chemisch und wirkungsmäßig die am nächsten verwandten Verbindungen sind. Die erfindungsgemäßen Wirkstoffe stellen somit eine Bereicherung der Technik dar.

Die erfindungsgemäßen substituierten Alkanyl-azolyl-oximcarbamate sind durch die Formel (I) allgemein definiert. In dieser Formel steht R¹ vorzugsweise für Wasserstoff und geradkettiges oder verzweigtes Alkyl mit 1 bis 4 Kohlenstoffatomen. R² steht vorzugsweise für Wasserstoff, geradkettiges oder verzweigtes Alkyl mit 1 bis 12 Kohlenstoffatomen, Allyl, ferner vorzugsweise für Halogenalkyl mit bis zu 2 Kohlenstoff- und bis zu 5 Halogenatomen, wie insbesondere Fluor- und Chloratomen sowie vorzugsweise für Alkoxyalkyl mit bis zu 2 Kohlenstoffatomen in jedem Alkylteil. R² steht außerdem vorzugsweise für die Gruppe -(S)mR³, falls R¹ für Alkyl steht. Dabei steht R³ vorzugsweise für geradkettiges oder verzweigtes Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, beispielsweise sei Trifluormethyl genannt, gegebenenfalls durch Chlor oder Trifluormethyl substituiertes Phenyl, sowie vorzugsweise für Alkoxycarbonyl mit 1 bis 4 Kohlenstoffatomen im Alkoxyteil. R³ steht ferner vorzugsweise für den gleichen Rest, an den die Gruppe - (S)m - R³ gebunden ist sowie für die Gruppe -NR⁴R⁵. R⁴ steht vorzugsweise für geradkettiges oder verzweigtes Alkyl mit 1 -4 Kohlenstoffatomen, R⁵ steht vorzugsweise für geradkettiges oder verzweigtes Alkyl mit 1-4 Kohlenstoffatomen, Dialkylcarbamoyl mit 1 bis 4 Kohfenstoffatomen in jedem Alkylteil, Alkoxycarbonyl mit 1 bis 4 Kohlenstoffatomen im Alkylteil sowie gegebenenfalls durch Halogen, insbesondere Fluor, Chlor oder Brom, Alkyl mit 1 bis 2 Kohlenstoffatomen, Halogenalkyl mit 1 bis 2 Kohlenstoff- und bis zu 5 Halogenatomen, wie insbesondere Fluor- und Chloratomen, beispielsweise Trifluormethyl substituiertes Phenylsulfonyl. R⁴ und R⁵ stehen außerdem gemeinsam mit dem Stickstoffatom noch vorzugsweise für Piperidino oder Morpholino. Die Symbole Az, X, m und n haben die in der Erfindungsdefinition angegebene Bedeutung.

Ganz besonders bevorzugt sind diejenigen substituierten Alkanyl-azolyl-oximcarbamate der Formel (I) in denen Az die in der Erfindungsdefinition angegebene Bedeutung hat, R¹ für Wasserstoff oder Methyl steht; R² für Methyl, Methoxymethyl oder Allyl sowie die Gruppe -(S)m-R³ steht; R³ für Methoxycarbonyl, Trichlormethyl, Dichlorfluormethyl, gegebenenfalls durch Chlor oder Trifluormethyl substituiertes Phenyl und die Gruppe -NR⁴R⁵ steht, wobei m für 1 steht; oder R³ den gleichen Rest bedeutet, an den die Gruppe - (S)m - R³ gebunden ist, wobei m für 1 oder 2 steht; R⁴ für Methyl steht, R⁵ für Methyl, Methoxycarbonyl und Methylphenylsulfonyl steht; R⁴ und R⁵ gemeinsam für Piperidino oder Morpholino stehen; X für gegebenenfalls einfach oder zweifach substituiertes tertiäres Butyl steht, wobei als Substituenten genannt seien: Chlor, Fluor, Brom, Hydroxy, Acetyloxy, Methylcarbamoyloxy und Dimethylcarbamoyloxy; n für 0 oder 1 steht.

Im Einzelnen seien außer den Herstellungsbeispielen und den Beispielen der Tabelle 1 die folgenden Verbindungen genannt:

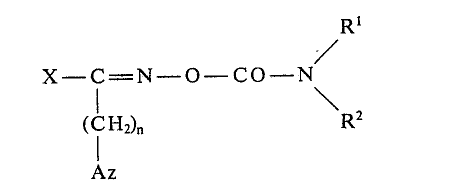

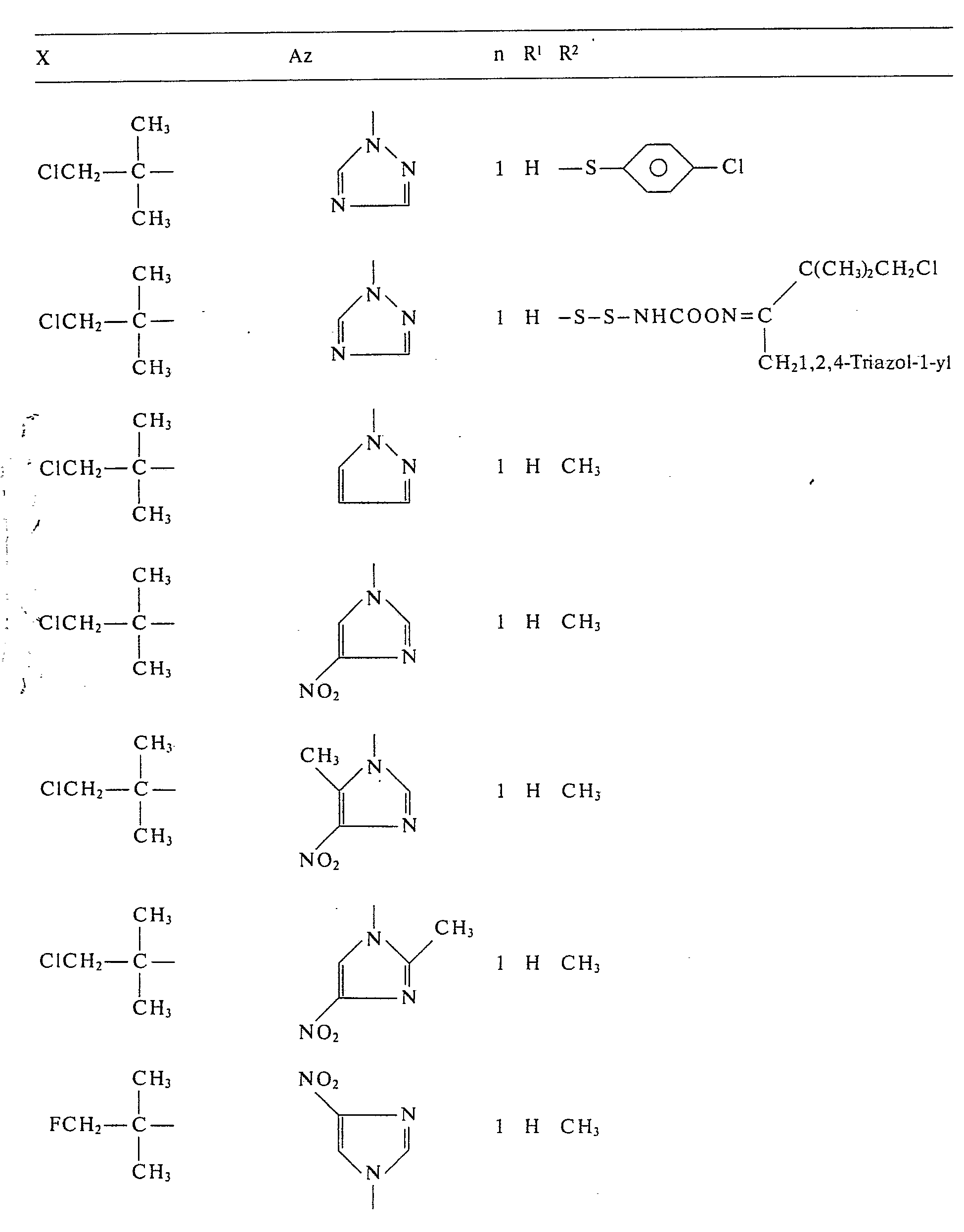

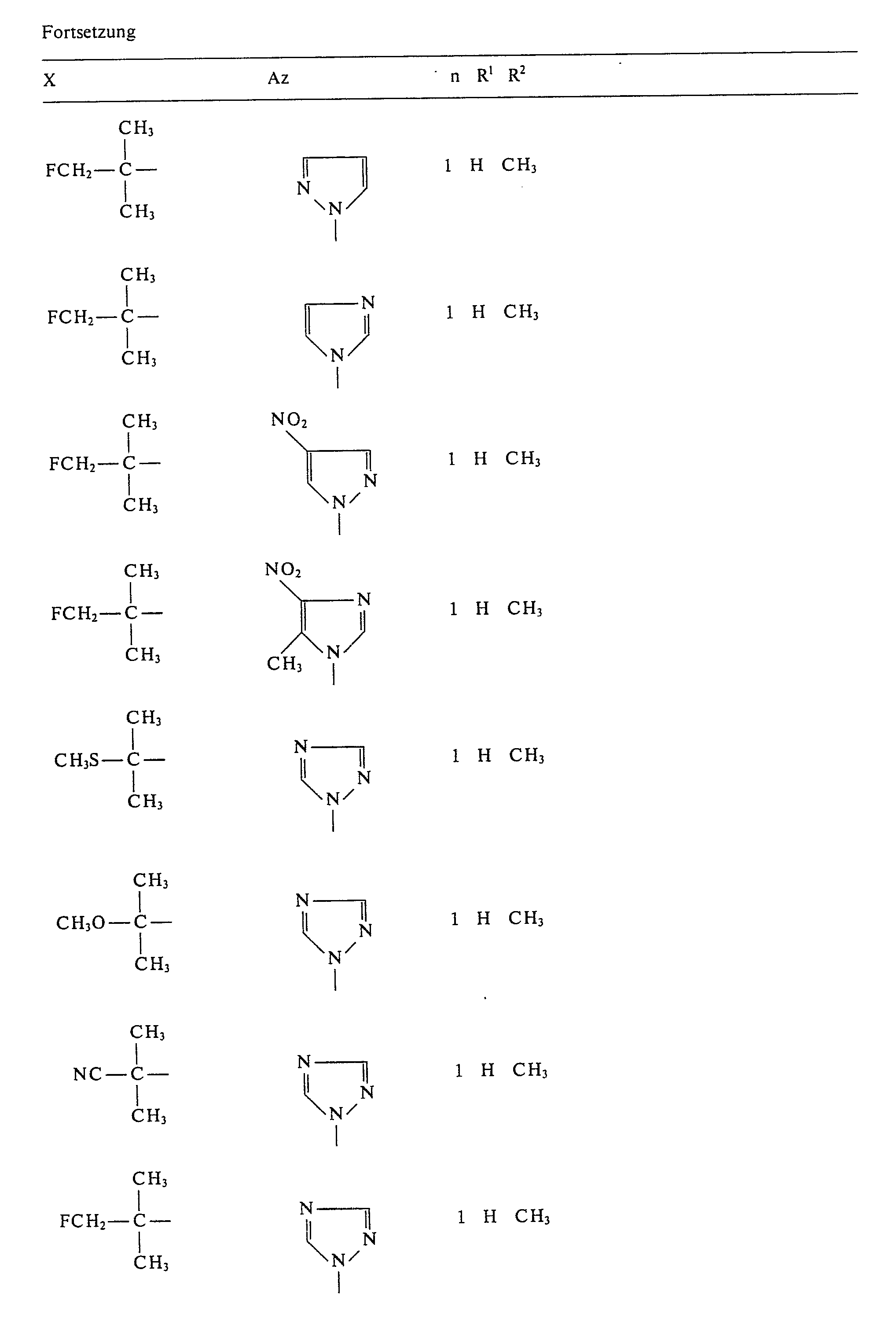

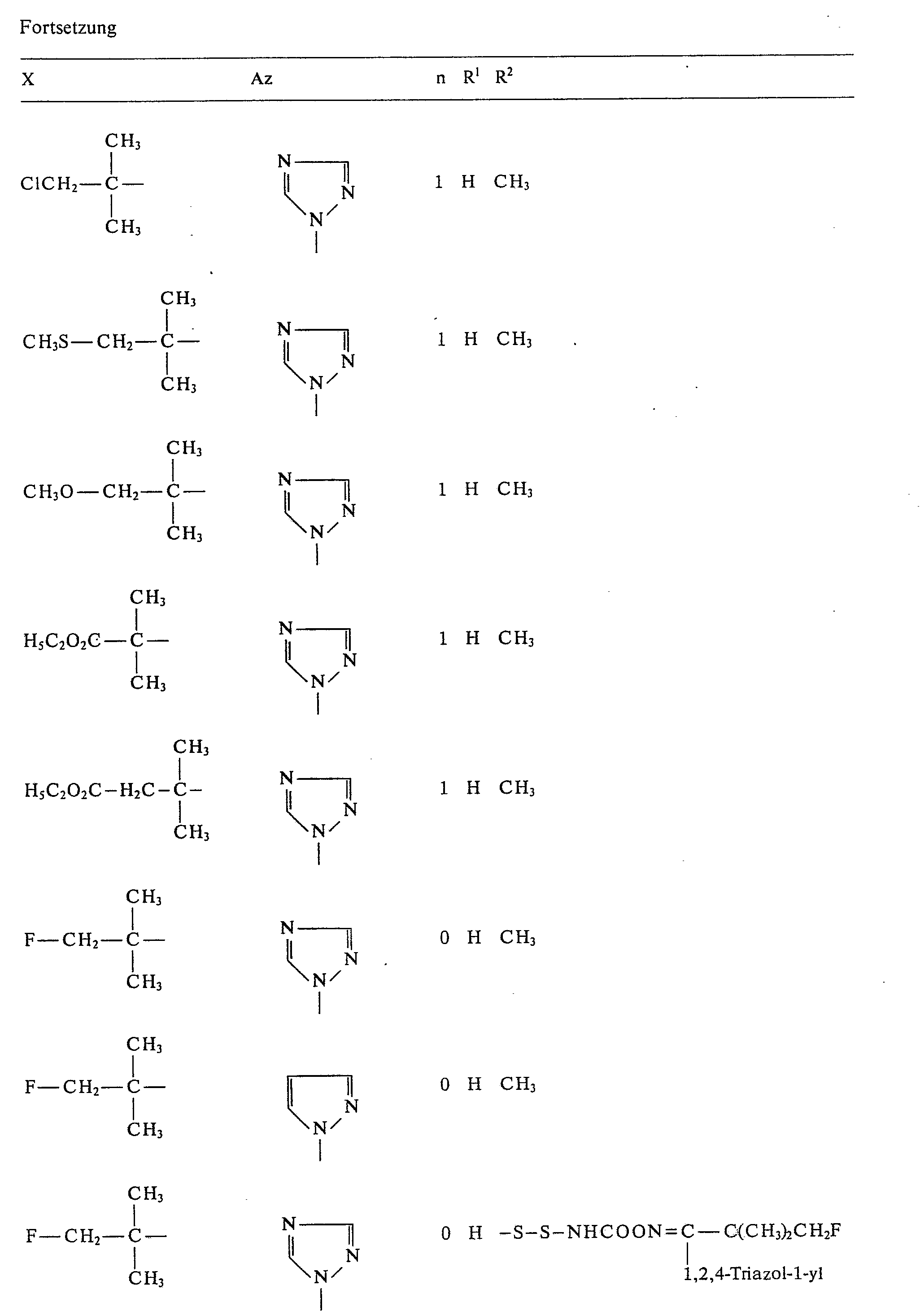

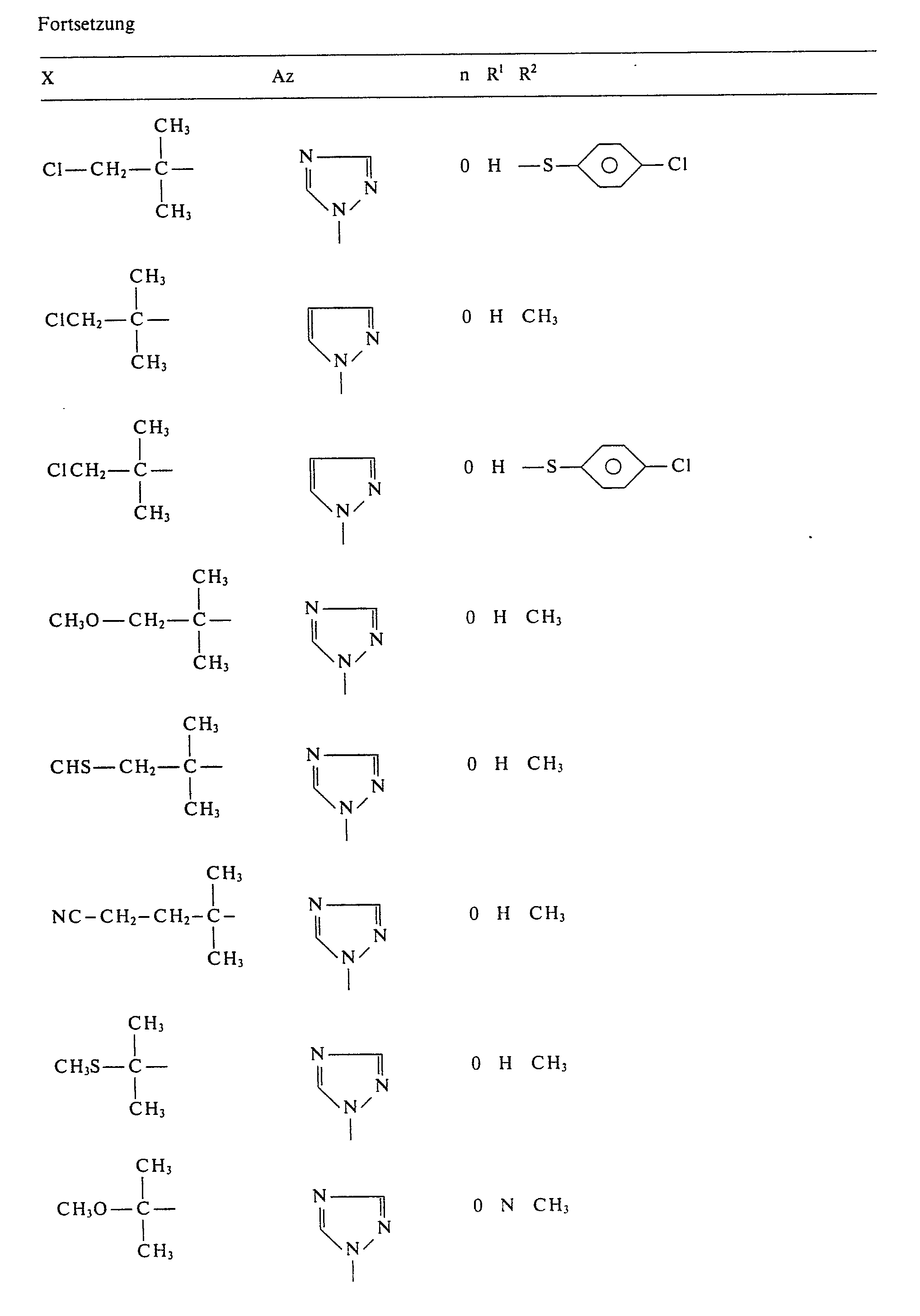

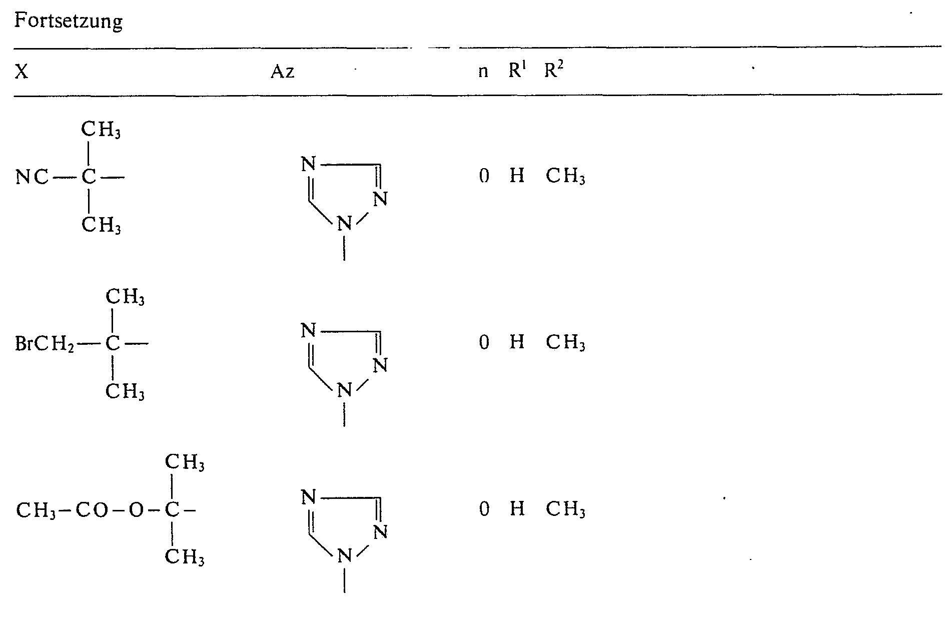

Verwendet man beispielsweise 4-Chlor-3,3-dimethyl-2-oximino-1-(1,2,4-triazol-1-yl)-butan und Dimethylcarbamoylchlorid als Ausgangsstoffe, so kann der Reaktionsablauf durch das folgende Formelschema wiedergegeben werden (Verfahren a):

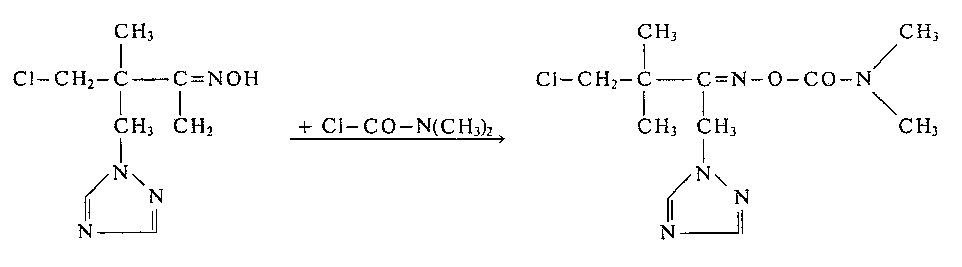

Verwendet man beispielsweise 4-Chlor-3,3-dimethyl-2-oximino-1-(pyrazol-1-yl)-butan und N-Methyl-N-trichlormethylmercaptocarbamoylfluorid als Ausgangsstoffe, so kann der Reaktionsablauf durch das folgende Formelschema wiedergegeben werden (Verfahren a):

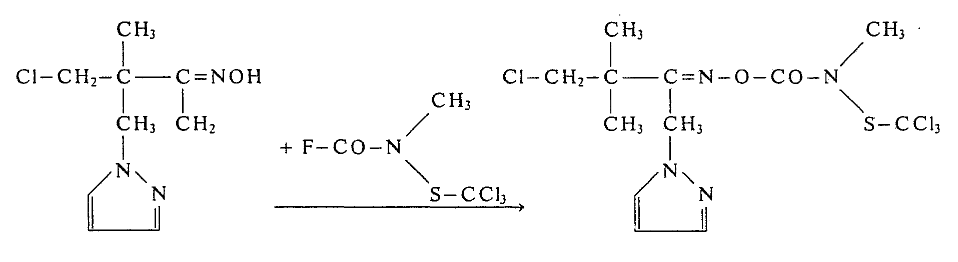

Verwendet man beispielsweise 3-Acetoxy-2,2-dimethyl-1-oximino-1-(1,2,4-triazol-1-yl)-propan und N,N'-Bis-(fluorcarbonyl)-thio-bis-methylamin als Ausgangsstoffe, so kann der Reaktionsablauf durch das folgende Formelschema wiedergegeben werden (Verfahren a):

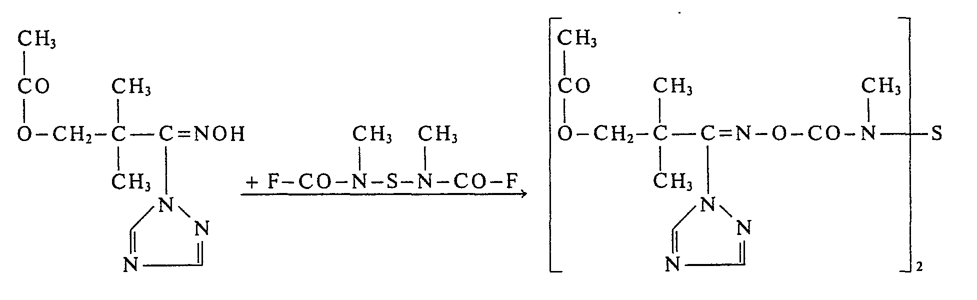

Verwendet man 3,3-Dimethyl-4-hydroxy-2-oximino-1-(1,2,4-triazol-7-yl)-butan und Methylisocyanat als Ausgangsstoffe, so kann der Reaktionsablauf durch das folgende Formelschema wiedergegeben werden (Verfahren b):

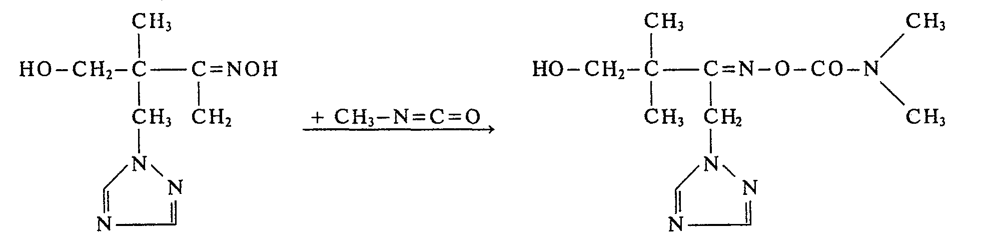

Verwendet man 4-Chlor-3,3-dimethyl-1-(imidazol-1-yl)-2-oximino-butan, Phosgen und Dimethylamin als Ausgangsstoffe, so kann der Reaktionsablauf durch das folgende Formelschema wiedergegeben werden (Verfahren c):

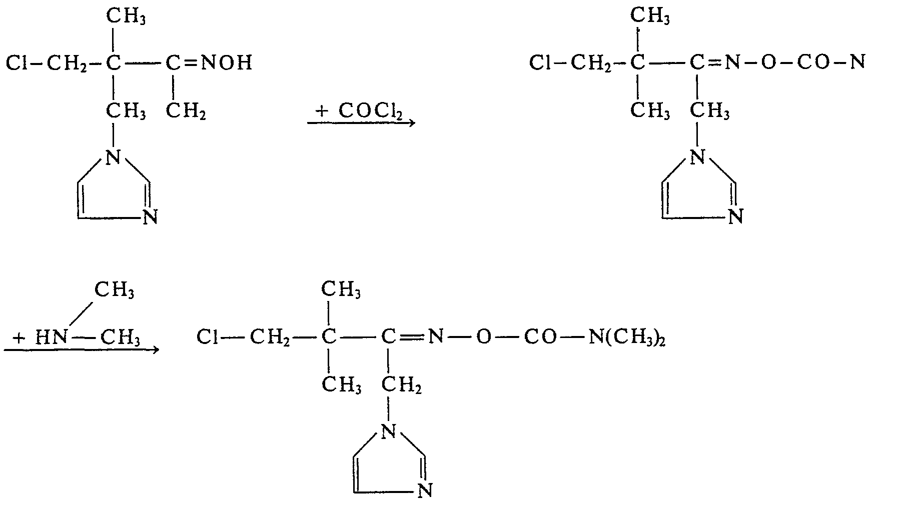

Verwendet man 2,2-Dimethyl-3-Fluor-1-methylcarbamoyloximino-1-(pyrazol-1-yl)-propan und 4-Chlorphenyl-sulfenylchlorid als Ausgangsstoffe, so kann der Reaktionsablauf durch das folgende

Formelschema wiedergegeben werden (Verfahren d):

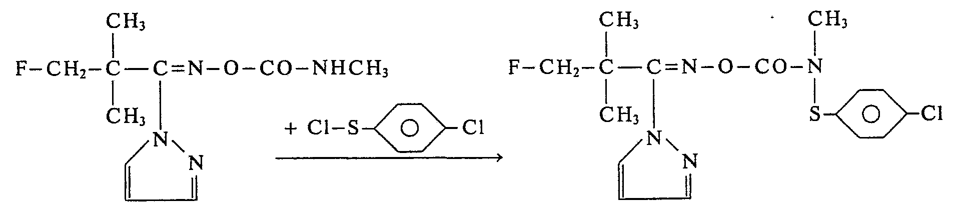

Die für die Verfahren (a), (b) und (c) als Ausgangsstoffe zu verwendenden Oxime sind durch die Formel (II) allgemein definiert. In dieser Formel stehen Az, X und n vorzugsweise für die Reste, die bei den Verbindungen der Formel (1) bereits vorzugsweise genannt wurden.

Die Oxime der Formel (II) sind noch nicht bekannt, können jedoch auf bekannte Art und Weise erhalten werden (vergleiche hierzu DE-OS 2 635 883).

Die Oxime der Formel (II), in denen n für 0 steht, können hergestellt werden, indem man Hydroxamsäurehalogenide der Formel (VIII) 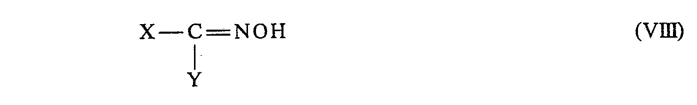 in welcher
X die oben angegebene Bedeutung hat und
Y für Halogen, insbesondere Chlor oder Brom steht,
mit Azolen der Formel (IX) 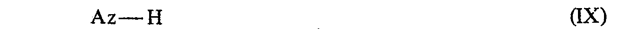
   in welcher

Az die oben angegebene Bedeutung hat,

in Gegenwart eines organischen Lösungsmittels, beispielsweise Tetrahydrofuran, und in Gegenwart eines Säurebinders, beispielsweise Trimethylamin oder überschüssiges Azol, bei Temperaturen zwischen 0 und 80° C, vorzugsweise 0 und 40° C, umsetzt. Die Isolierung der Verbindungen der Formel (II) erfolgt dadurch, daß man das Reaktionsgemisch mit Wasser versetzt, den entstehenden Niederschlag abfiltriert, trocknet und gegebenenfalls durch Umkristallisation reinigt.

Die als Ausgangsstoffe verwendeten Hydroxamsäurehalogenide der Formel (VIII) sind bekannt (vgl. H. Ulrich »The Chemistry of Imidoyl Halides«, Seiten 157-172, Plenum Press, New York 1968 und die dort zitierten Literaturstellen). Noch nicht bekannte lassen sich nach den dort beschriebenen Verfahren leicht herstellen, so z. B. durch Chlorierung-der entsprechenden Aldoxime.

Die Oxime der Formel (11), in denen n für 1 steht, können hergestellt werden, indem man Azolyiketone der Formel 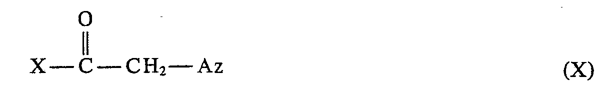 in welcher
Az und X die oben angegebene Bedeutung haben,

mit Hydroxylamin in Gegenwart eines Lösungsmittels, vorzugsweise Alkohole bzw. wäßrige Alkohole, bei Temperaturen zwischen 20 und 100°C, vorzugsweise zwischen 50 und 80° C, umsetzt. Dabei wird das Hydroxylamin vorzugsweise in Form seiner Salze, insbesondere als Hydrochlorid, in Gegenwart eines Säurebindemittels, wie beispielsweise Natriumcarbonat, eingesetzt. Die Isolierung der Verbindungen der Formel (II) erfolgt dadurch, daß man das während der Umsetzung entstehende Produkt nach Abdestillieren des Lösungsmittels nach üblichen Methoden aufarbeitet.

Die Azolylketone der Formel (X) können erhalten werden, indem man Halogenketone der Formel (XI) 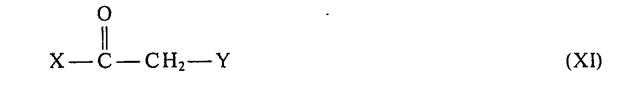 in welcher
X die oben angegebene Bedeutung hat und
Y für Chlor oder Brom steht,

mit Azolen der Formel (IX) in Gegenwart eines Verdünnungsmittels, beispielsweise Methyläthylketon, und in Gegenwart eines Säurebindemittels, beispielsweise Kaliumcarbonat, bei Temperaturen zwischen 20 bis 150°C, vorzugsweise zwischen 60 bis 120° C, umsetzt. Die Isolierung der Verbindungen der Formel (XI) erfolgt dadurch, daß man das während der Reaktion entstehende Salz abfiltriert und das Filtrat durch Abdestillieren des Lösungsmittels einengt. Der dabei zurückbleibende Feststoff wird getrocknet und durch Umkristallisation gereinigt.

Als Ausgangsstoffe der Formel (11) seien beispielsweise genannt:
4-Chlor-3,3-dimethyl-2-oximino-1-(1,2,4-triazol-1-yl)-butan
4-Chlor-3,3-dimethyl-2-oximino-1-(pyrazol-1-yl)-butan
4-Chlor-3,3-dimethyl-2-oximino-1 -(imidazol-1 -yl)-butan
4-Chlor-3,3-dimethyl-2-oximino-1-(5-methyl-4-nitro-imidazol-1-yl)-butan
4-Chlor-3,3-dimethyl-2-oximino-1-(4-nitro-imidazol-1-yl)-butan
4-Chlor-3,3-dimethyl-2-oximino-1-(2-methyl-4-nitro-imidazol-1-yl)-butan
4-Methylthio-3,3-dimethyl-2-oximino-1-(1,2,4-triazol-1-yl)-butan
4-Methylthio-3,3-dimethyl-2-oximino-1-(pyrazol-1-yl)-butan
4-Methylthio-3,3-dimethyl-2-oximino-1-(imidazol-1-yl)-butan
4-Methylthio-3,3-dimethyl-2-oximino-1-(5-methyl-4-nitro-imidazol-1-yl)-butan
4-Methylthio-3,3-dimethyl-2-oximino-1-(4-nitro-imidazol-1-yl)-butan
4-Methylthio-3,3-dimethyl-2-oximino-1-(2-methyl-4-nitro-imidazol-1-yl)-butan
4-Fluor-3,3-dimethyl-2-oximino-1-(1,2,4-triazol-1-yl)-butan
4-Fluor-3,3-dimethyl-2-oximino-1-(pyrazol-1-yl)-butan
4-Fluor-3,3-dimethyl-2-oximino-1-(imidazol-1-yl)-butan
4-Fluor-3,3-dimethyl-2-oximino-1-(5-methyl-4-nitro-imidazol-1-yl)-butan
4-Fluor-3,3-dimethyl-2-oximino-1-(4-nitro-imidazol-1-yl)-butan
4-Fluor-3,3-dimethyl-2-oximino-1-(2-methyl-4-nitro-imidazol-1-yl)-butan
4-Methoxy-3,3-dimethyi-2-oximino-1-(1,2,4-triazol-1-yl)-butan
4-Methoxy-3,3-dimethyl-2-oximino-1-(pyrazol-1-yl)-butan
4-Methoxy-3,3-dimethyl-2-oximino-1-(imidazol-1-yl)-butan
4-Methoxy-3,3-dimethyl-2-oximino-1-(5-methyl-4-nitro-imidazol-1-yl)-butan
4-Methoxy-3,3-dimethyl-2-oximino-1-(4-nitro-imidazol-1-y)-butan
4-Methoxy-3,3-dimethyl-2-oximino-1-(2-methyl-4-nitro-imidazol-1-yl)-butan
2-Methyl-2-methoxy-1-oximino-1-(1,2,4-triazoi-l-yl)-propan
2-Methyl-2-methoxy-1-oximino-1-(pyrazol-1-yl)-propan
2-Methyl-2-methoxy-1-oximino-1-(imidazol-1-yl)-propan
2-Methyl-2-methylthio-1-oximino-1-(1,2,4-triazol-1-yl)-propan
2-Methyl-2-methylthio-1-oximino-1-(pyrazol-1-yl)-propa
2-Methyl-2-methylthio-1-oximino-1-(imidazol-1-yl)-propan
2-Methyl-2-acetoxy-1-oximino-1-(1,2,4-trizaol-1-yl)-propan
2-Methyl-2-acetoxy-1-oximino-1-(pyrazol-1-yl)-propan
2-Methyl-2-acetoxy-1-oximino-1-(imidazol-1-yl)-propan
2-Methyl-2-cyano-1-oximino-1-(1,2,4-triza ol-1-yl)-propan
2-Methyl-2-cyano-1-oximino-(1-(pyrazol-1-yl)-propan
2-Methyl-2-cyano-1-oximino-1-(imidazol-1-yl)-propan
3-Chlor-2,2-dimethyl-1-oximino-1-(1,2,4-triazol-1-yl)-propan
3-Chlor-2,2-dimethyl-1-oximino-1-(pyrazol-1-yl)-propan
3-Chlor-2,2-dimethyl-1-oximino-1-(imidazol-1-yl)-propan
3-Fluor-2,2-dimethyl-1-oximino-1-(1,2,4-triazol-1-yl)-propan
3-Fluor-2,2-dimethyl-1-oximino-1-(pyrazol-1-yl)-propan
3-Fluor-2,2-dimethyl-1-oximino-1-(imidazol-1-yl)-propan
3-Methylthio-2,2-dimethyl-1-oximino-1-(1,2,4-triazol-1-yl)-propan
3-Methylthio-2,2-dimethyl-1-oximino-1-(pyrazol-1-yl)-propan
3-Methylthio-2,2-dimethyl-1-oximino-1-(imidazol-1-yl)-propan
3-Methoxy-2,2-dimethyl-1-oximino-1-(1,2,4-triazol-1-yl)-propan.
3-Methoxy-2,2-dimethyl-1-oximino-1-(pyrazol-1-yl)-propan
3-Methoxy-2,2-dimethyl-1-oximino-1-(imidazol-1-yl)-propan

Die außerdem für die erfindungsgemäße Umsetzung (a) als Ausgangsstoffe benötigten Carbamoylhalogenide sind durch die Formel (III) allgemein definiert. In dieser Formel stehen R¹ und R² vorzugsweise für die Reste, die bei den Verbindungen der Formel (I) bereits vorzugsweise genannt wurden.

Die Carbamoylhalogenide der Formel (III) sind bekannt oder lassen sich nach allgemein üblichen und bekannten Verfahren herstellen, z. B. erhält man sie durch Umsetzung von Aminen mit Phosgen (diese Verfahren sind aus den allgemeinen Lehrbüchern der organischen Chemie bekannt) oder durch Umsetzung der entsprechenden Carbamidsäurehalogenide mit entsprechenden Sulfenchloriden (vergleiche hierzu auch die Angaben in DE-AS 1 297095, DE-OS 2 357 930 und 2 409 463 sowie US-Patentschrift 3 939 192).

Als Ausgangsstoffe der Formel (III) seien beispielsweise genannt:
Dimethylcarbamoylchlorid
Methyläthylcarbamoylchlorid
Allylmethylcarbamoylchlorid
Methoxymethyl-methylcarbamoylchlorid
Methyl-trifluormethylcarbamoylchlorid
N,N'-Bis-(fluorcarbonyl)-thio-bis-methylamin
N-Methyl-N-trichlormethylsulfenyl-carbamidsäurefluorid
N-Methyl-N-fluor-dichlormethyisulfenyl-carbamidsäurefluorid
N-Methyl-N-chlor-difluormethylsulfenyl-carbamidsäurefluorid
N-Methyl-N-(3-trifluormethylphenyl)-sulfenyl-carbamidsäurefluorid
N-Methyl-N-(methoxycarbonyl-sulfenyl)-carbamidsäurefluorid
N-Methyl-N-[(3-methylphenyl-sulfonyl)-methylamino-sulfenyl)]-carbamidsäurefluorid
N-Methyl-N-[(4-chlorphenyl)-sulfenyl]-carbamidsäurefluorid
N-Methyl-N-[(4-methylphenyl-sulfonyl)-methylamino-sulfenyl]-carbamidsäurefluorid
N-Methyl-N-(morpholin-1-yl-sulfenyl)-carbamidsäurefluorid

und die entsprechenden Carbamidsäure-chloride.

Die weiterhin für die erfindungsgemäße Umsetzung (b) als Ausgangsstoffe benötigten Isocyanate sind durch die Formel (IV) allgemein definiert. In dieser Formel steht R vorzugsweise für geradkettiges oder verzweigtes Alkyl mit 1 bis 12 Kohlenstoffatomen, Allyl, für Alkoxyalkyl mit bis zu 2 Kohlenstoffatomen in jedem Alkylteil.

Die lsocyanate der Formel (IV) sind bekannt oder lassen sich nach allgemein üblichen und bekannten Verfahren herstellen, z. B. durch Umsetzung von Aminen mit Phosgen und anschließendem Erhitzen. Diese Verfahren sind aus den allgemeinen Lehrbüchern der organischen Chemie bekannt.

Als Ausgangsstoffe der Formel (IV) seien beispielsweise genannt:
Methoxymethylisocyanat, Äthoxymethylisocyanat, Methoxyäthylisocyanat, Methylisocyanat, Äthylisocyanat, i-Propylisocyanat, t-Butylisocyanat, Heptylisocyanat, Dodecylisocyanat, Allylisocyanat.

Die weiterhin für die erfindungsgemäße Umsetzung (c) als Ausgangsstoffe zu verwendenden Amine sind durch die Formel (V) allgemein definiert. In dieser Formel steht R vorzugsweise für Wasserstoff sowie die Reste, die bei den Isocyanaten der Formel (IV) bereits vorzugsweise genannt wurden. R^{l} steht vorzugsweise für die Reste, die bei den Verbindungen der Formel (I) bereits vorzugsweise genannt wurden.

Die Amine der Formel (V) sind allgemein bekannte Verbindungen. Als Beispiele seien genannt:

Ammoniak, Methylamin, Äthylamin, Dimethylamin, Methyläthylamin, Allylmethylamin, Methoxymethyl-methylamin.

Die für die erfindungsgemäße Umsetzung (d) als Ausgangsstoffe zu verwendenden Sulfenchloride sind durch die Formel (VII) allgemein definiert. In dieser Formel steht R³ vorzugsweise für die Reste, die bei den Verbindungen der Formel (I) bereits vorzugsweise genannt wurden.

Die Sulfenchloride der Formel (VII) sind allgemein bekannte Verbindungen der organischen Chemie. Als Beispiele seien genannt:
Trichlormethylsulfenchlorid
Dichlorfluormethylsulfenchlorid
Chlordifluormethylsulfenchlorid
Trifluormethylsulfenchlorid
Phenylsulfenchlorid
2,4-Dichlorphenylsulfenchlorid
3-Trifluormethylsuifenchlorid
3-Methylphenylsulfenchlorid
Methylsulfenylchlorid
4-Chlor-3-trifluor-methylphenylsulfenylchlorid
Methoxycarbonylsulfenylchlorid
Äthoxycarbonylsulfenylchlorid

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäure, wie z. B. p-Toluolsulfonsäure und 1,5-Naphthatindisuifonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (1) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seinen. Als Anionen der Salze kommen solche in Betracht, die sich von physiologischen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure. Die Metallsalzkomplexe der Verbindungen der Formel (1) können in einfacher Weise nach üblichen Verfahren erhalten werden, so z. B. durch Lösen des Metallsalzes in Alkohol, z. B. Äthanol, und Hinzufügen zur Verbindung der Formel (I). Man kann Metallsalzkomplexe in bekannter Weise, z. B. durch Abfiltrieren, Isolieren und gegebenenfalls durch Umkristallisation reinigen.

Als Verdünnungsmittel kommen für die Umsetzung gemäß Verfahrensvariante (a), (b), (c) und (d) vorzugsweise alle inerten organischen Lösungsmittel infrage. Hierzu gehören vorzugsweise Ketone, wie Diäthylketon, insbesondere Aceton und Methyläthylketon; Nitrile, wie Propionitril, insbesondere Acetonitril; Alkohole, wie Äthanol oder lsopropanol; Äther, wie Tetrahydrofuran oder Dioxan; Formamide, wie insbesondere Dimethylformamid und halogenierte Kohlenwasserstoffe, wie Methylenchlorid, Tetrachlorkohlenstoff oder Chloroform. Bei Verwendung von Natriumhydrid als Hilfsstoff werden vorzugsweise polare organische Lösungsmittel verwendet, wie insbesondere Hexamethylphosphorsäuretriamid.

Wird die Umsetzung nach Verfahren (a), (c) und (d) in Gegenwart eines Säurebinders vorgenommen, so kann man alle üblicherweise verwendbaren anorganischen und organischen Säurebinder zugeben. Hierzu gehören vorzugsweise Natriumcarbonat, Kaliumcarbonat und Natriumhydrogencarbonat, ferner niedere tertiäre Alkylamine, Cycloalkylamine oder Arylalkylamine, wie beispielsweise Triäthylamin, N,N-Dimethyl-benzylamin, Dicyclohexylamin, weiterhin Pyridin und Diazabicylooctan.

Die Reaktionstemperaturen können bei der Durchführung des Verfahren (a) in einem größeren Bereich variiert werden. Im allgemeinen arbeitet man zwischen 0 und 100° C, vorzugsweise zwischen 10 und 80° C.

Bei der Durchführung des Verfahrens (a) setzt man vorzugsweise auf 1 Mol der Verbindung der Formel (II) 1 bis 2 Mol, für den Fall eines dimeren Produktes 0,5 Mol, an Carbamoylchlorid der Formel (III) und 1 bis 2 Moi Säurebinder ein. Die Isolierung der Verbindungen der Formel (1) erfolgt in allgemein üblicher und bekannter Weise.

Als Katalysatoren können beim Verfahren (b) vorzugsweise verwendet werden:
tertiäre Basen, wie Triäthylamin, Pyridin, Zinn-organische Verbindungen, wie Dibutylzinndilaurat.

Die Reaktionstemperaturen können bei der Durchführung des Verfahren (b) in einem größeren Bereich variiert werden. Im allgemeinen arbeitet man zwischen 0 und 100° C, vorzugsweise zwischen 20 und 85° C.

Bei der Durchführung des Verfahrens (b) setzt man auf 1 Mol der Verbindung der Formel (11) 1 bis 2 Mol Isocyanat der Formel (IV) ein. Zur Isolierung der Verbindungen der Formel (I) wird das Lösungsmittel abdestilliert und der Rückstand nach üblichen Methoden aufgearbeitet.

Die Reaktionstemperaturen können bei der Durchführung des Verfahrens (c) in einem größeren Bereich variiert werden. Im allgemeinen arbeitet man zwischen 0 und 100° C, vorzugsweise zwischen 0 und 85° C.

Bei der Durchführung des Verfahrens (c) setzt man vorzugsweise auf 1 Mol der Verbindung der Formel (11) 1 bis 1,5 Mol Phosgen und 1 bis 1,5 Mol an Amin der Formel (V) ein. Es hat sich als vorteilhaft erwiesen, den Säurebinder in geringem Überschuß (bis ca. 30 Gewichtsprozent) und gegebenenfalls das Natriumhydrid in einem Überschuß bis zu ca. 50 Gewichtsprozent einzusetzen. Die Isolierung der Verbindungen der Formel (I) erfolgt in üblicher Weise.

Die Reaktionstemperaturen können bei der Durchführung des Verfahrens (d) in einem größeren Bereich variiert werden. Im allgemeinen arbeitet man zwischen 0 und 100°C, vorzugsweise zwischen 10 und 50° C.

Bei der Durchführung des Verfahrens (d) werden die Ausgangsstoffe vorzugsweise in molaren Mengen eingesetzt. Die Isolierung der Verbindungen der Formel (I) erfolgt nach übljchen Methoden. Zum Teil ist es auch möglich die einzelnen Stufen der Vorprodukte zur Herstellung der Oxime der Formel (II) sowie deren Umsetzung zu den erfindungsgemäßen Stoffen ohne Isolierung der jeweiligen Zwischenprodukte in einer sogenannten Eintopfreaktion durchzuführen.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophaus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips fermoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia. podana, Capua reticulana, Choristoneura fumiferana, Clysia ambiguella, Homona magnanima, Tortrix viridana.

Aus der Ordnung der Coleoptara z. B. Anobium punctatum, Rhizopertha dominica, Bruchidius obtectus, Acanthoscelides obxecctus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus suicatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinalla frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

### Beispiel A

### Doralis-Test (systemische Wirkung)

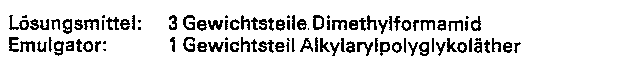

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung werden Bohnenpflanzen (Vicia faba), die stark von der schwarzen Bohnenlaus (Doralis fabae) befallen sind, angegossen, so daß die Wirkstoffzubereitung in den Boden eindringt, ohne die Blätter der Bohnenpflanzen zu benetzen. Der Wirkstoff wird von den Bohnenpflanzen aus dem Boden aufgenommen und gelangt so zu den befallenen Blättern.

Nach den angegebenen Zeiten wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z. B. die folgenden Verbindungen der Herstellungsbeispiele überlegene Wirkung gegenüber dem Stand der Technik: 7, und 2.

### Beispiel B

### Tetranychus-Test (resistent)

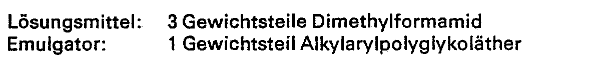

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 7 und 3.

### Beispiel C

### Grenzkonzentrationstest/Wurzelsystemische Wirkung

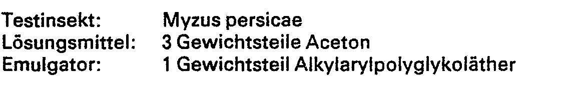

Zur Herstellung einer zweckmäßigen Wirkstoffzubereitung vermischt man 1 Gewichtsteil Wirkstoff -mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik: 3 und 7.

### Beispiel D

### Grenzkonzentrationstest/Wurzelsystemische Wirkung

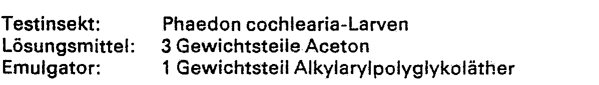

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik: 3 und 7.

### Beispiel E

### Grenzkonzentrations-Test

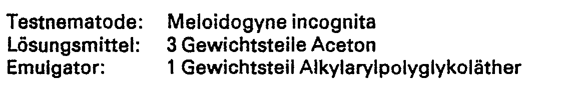

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27° C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z. B. die folgenden Verbindungen der Herstellungsbeispiele überlegene Wirksamkeit gegenüber dem Stand der Technik: 3 und 7.

### Herstellungsbeispiele

### Beispiel 1

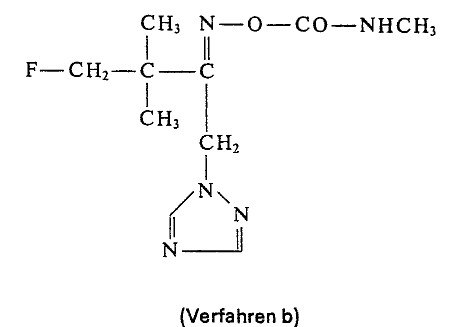

6g (0,03 Mol) 3,3-Dimethyl-4-fluor-2-oximino-1-(1,2,4-triazol-1-yl)-butan werden in 100 ml Methylenchlorid gelöst und unter Rühren mit 5 ml (0,06 Mol) Methylisocyanat versetzt. Man läßt 12 Stunden bei Raumtemperatur stehen und destilliert anschließend unter Vakuum die flüchtigen Anteile ab. Der ölige Rückstand wird durch Anreiben mit Petroläther zur Kristallisation gebracht. Man erhält 7 g (90% der Theorie) 3,3-Dimethyl-4-fluor-2-methylcarbamoyloximino-1-(1,2,4-triazol-1-yl)-butan vom Schmelzpunkt 78-79°C.

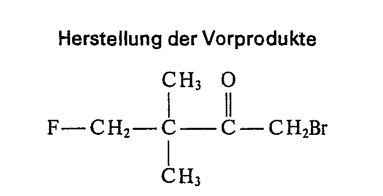

### 1. Stufe:

38 g (0,32 Mol) 3,3-Dimethyl-4-fluor-butan-2-on werden in 250 ml Äther gelöst und bei 20° C unter Kühlung tropfenweise mit 52 g (0,325 Mol) Brom versetzt. Man läßt 1 Stunde nachrühren, wäscht die ätherische Lösung fünfmal mit je 100 ml Wasser, trocknet über Natriumsulfat und engt im Vakuum ein. Das resultierende 1-Brom-3,3-dimethyl-4-fluor-butan-2-on wird ohne weitere Reinigung weiter umgesetzt:

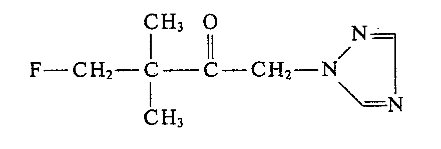

### 2. Stufe

Eine Mischung aus 23,1 g (0,33 Mol) 1,2,4-Triazol und 46,2 g (0,4 Mol) Kaliumcarbonat in 250 ml Aceton wird bei Raumtemperatur unter Kühlung tropfenweise mit einer Lösung des in der 1. Stufe erhaltenen 1-Brom-3,3-dimethyl-4-fluor-butan-2-ons in 50 ml Aceton versetzt. Man läßt 4 Stunden bei 20° C nachrühren, saugt vom anorganischen Niederschlag ab und engt das Filtrat im Vakuum ein. Das resultierende ölige 3,3-Dimethyl-4-fluor-1-(1 ,2,4-triazol-1-yl)-butan-2-on wird ohne weitere Reinigung weiter umgesetzt:

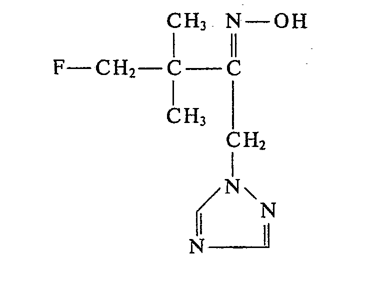

### 3. Stufe

Das in der 2. Stufe erhaltene 3,3-Dimethyl-4-fluor-1-(1,2,4-triazol-1-yl)-butan-2-on sowie 42 g (0,6 Mol) Hydroxylaminhydrochlorid und 33 g (0,33 Mol) Triäthylamin werden in 250 ml Äthanol gelöst und 5 Stunden unter Rückfluß erhitzt. Danach engt man durch Abdestillieren des Lösungsmittels nahezu zur Trockne ein. Der Rückstand wird mit Wasser aufgenommen und die resultierenden Kristalle abgesaugt. Man erhält 24g (37,5% der Theorie, bezogen auf in der 1. Stufe eingesetztes 3,3-Dimethyl-4-fluor-butan-2-on) 3,3-Dimethyl-4-fluor-2-oximino-1-(1,2,4-triazol-1-yl)-butan vom Schmelzpunkt 124-126° C.

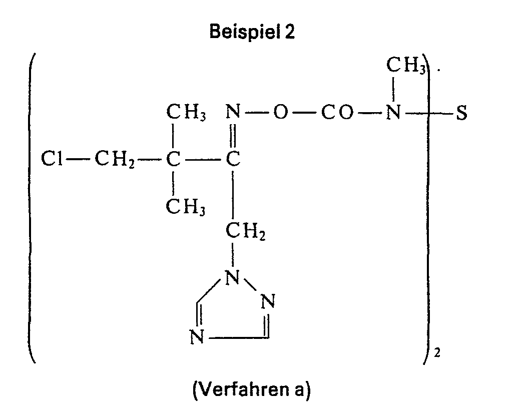

5 g (0,025 Mol) 3-Chlor-2,2-dimethyl-1-oximino-1-(1,2,4-triazol-1-yl)-propan und 2,3 g (0,0125 Mol) Thio-bis-(N-methyl-carbamidsäurefluorid) werden in 50 ml Dioxan gelöst und bei 20-25°C tropfenweise mit 2,5 g (0,025 Mol) Triäthylamin versetzt. Nach 12stündigem Stehen bei Raumtemperatur wird das Reaktionsgemisch mit 100 ml Wasser versetzt. Das ausgeschiedene Festprodukt wird abgesaugt, mit Wasser gewaschen und getrocknet. Man erhält 5 g (73% der Theorie) N,N'-Bis-[3-Chlor-2,2-dimethyl-1-oximino-carbonyl-1-(1,2,4-triazol-1-yl)-propan]-thio-bis-methylamin vom Schmelzpunkt 168-170°C.

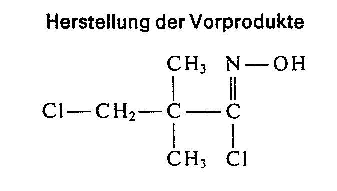

### 1. Stufe

45 g (0,37 Mol) 3-Chlor-2,2-dimethyl-propanol und 39 g (0,55 Mol) Hydroxylaminhydrochlorid werden in 200 ml Wasser eingerührt und tropfenweise mit einer Lösung von 29 g (0,27 Mol) Natriumcarbonat in 100 ml Wasser versetzt. Man läßt 5 Stunden bei Raumtemperatur rühren und stellt dann die Reaktionslösung mit 20 ml konzentrierter Salzsäure auf einem pH-Wert von 1. Danach werden bei 0-5°C 30 g Chlor eingeleitet. Anschließend trennt man die untere organische Phase ab und setzt das rohe 1,3-Dichlor-2,2-dimethyl-1-oximino-propan direkt weiter um:

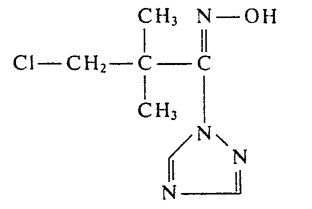

### 2. Stufe

30,6 g (0,44 Mol) 1,2,4-Triazol und 59 g (0,55 Mol) Natriumcarbonat werden in 300 ml Wasser gelöst und anschließend bei Raumtemperatur tropfenweise mit dem in der 1. Stufe erhaltenen rohen 1,3-Dichlor-2,2-dimethyl-1-oximino-propan versetzt. Man läßt 5 Stunden bei 20° C rühren und extrahiert mehrmals mit Methylenchlorid. Die vereinigten Methylenchloridphasen werden über Natriumsulfat getrocknet und im Vakuum eingeengt. Der Rückstand kristallisiert nach dem Verreiben mit Diisopropyläther. Man erhält 13 g (17,5% der Theorie, bezogen auf das in der 1. Stufe eingesetzte 3-Chlor-2,2-dimethyl-propanol) 3-Chlor-2,2-dimethyl-1-oximino-1-(1,2,4-triazot-1-yl)-propan vom Schmelzpunkt 148-153°C.

Analog werden die folgenden Beispiele der Tabelle 1 erhalten.

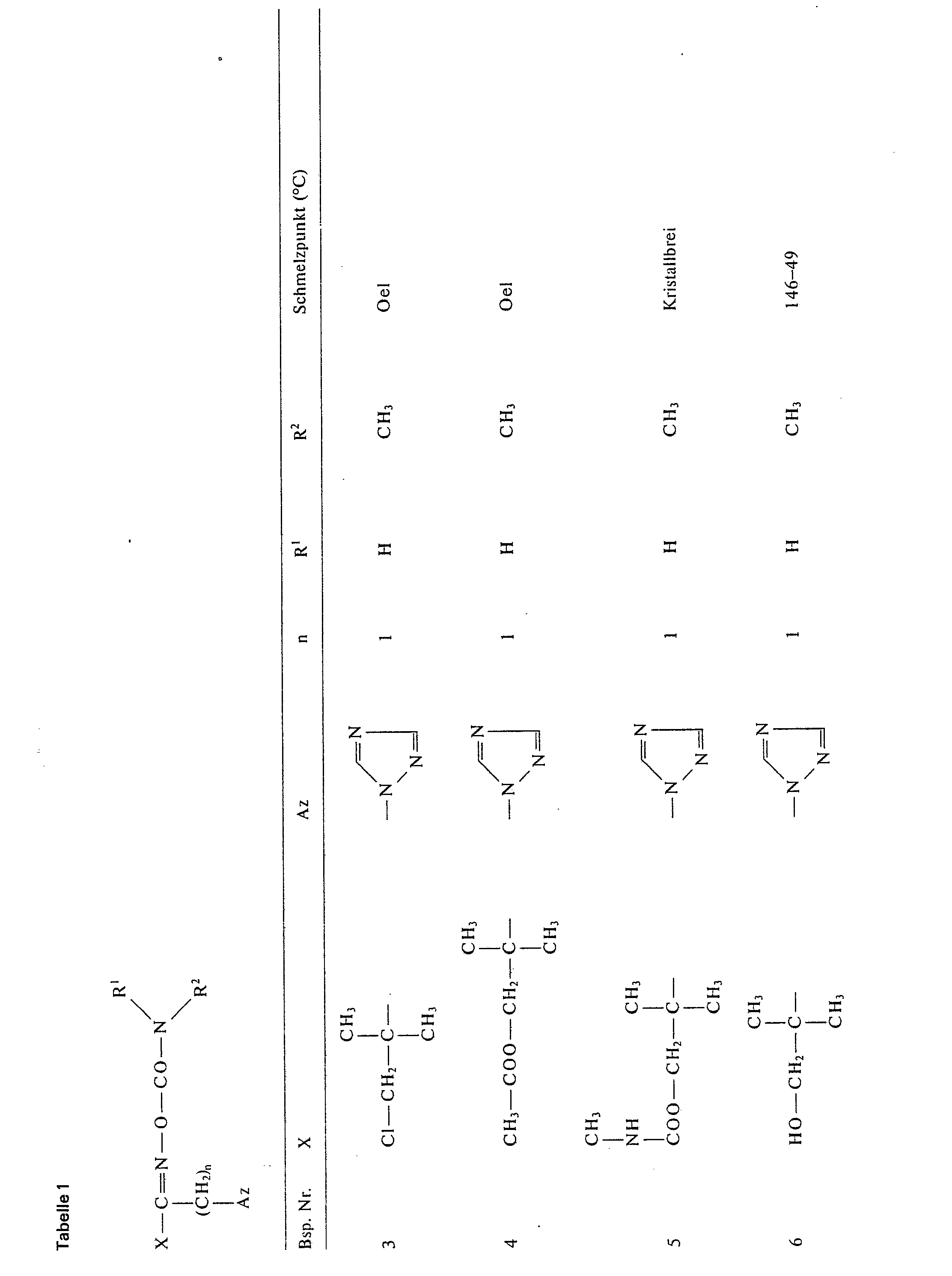

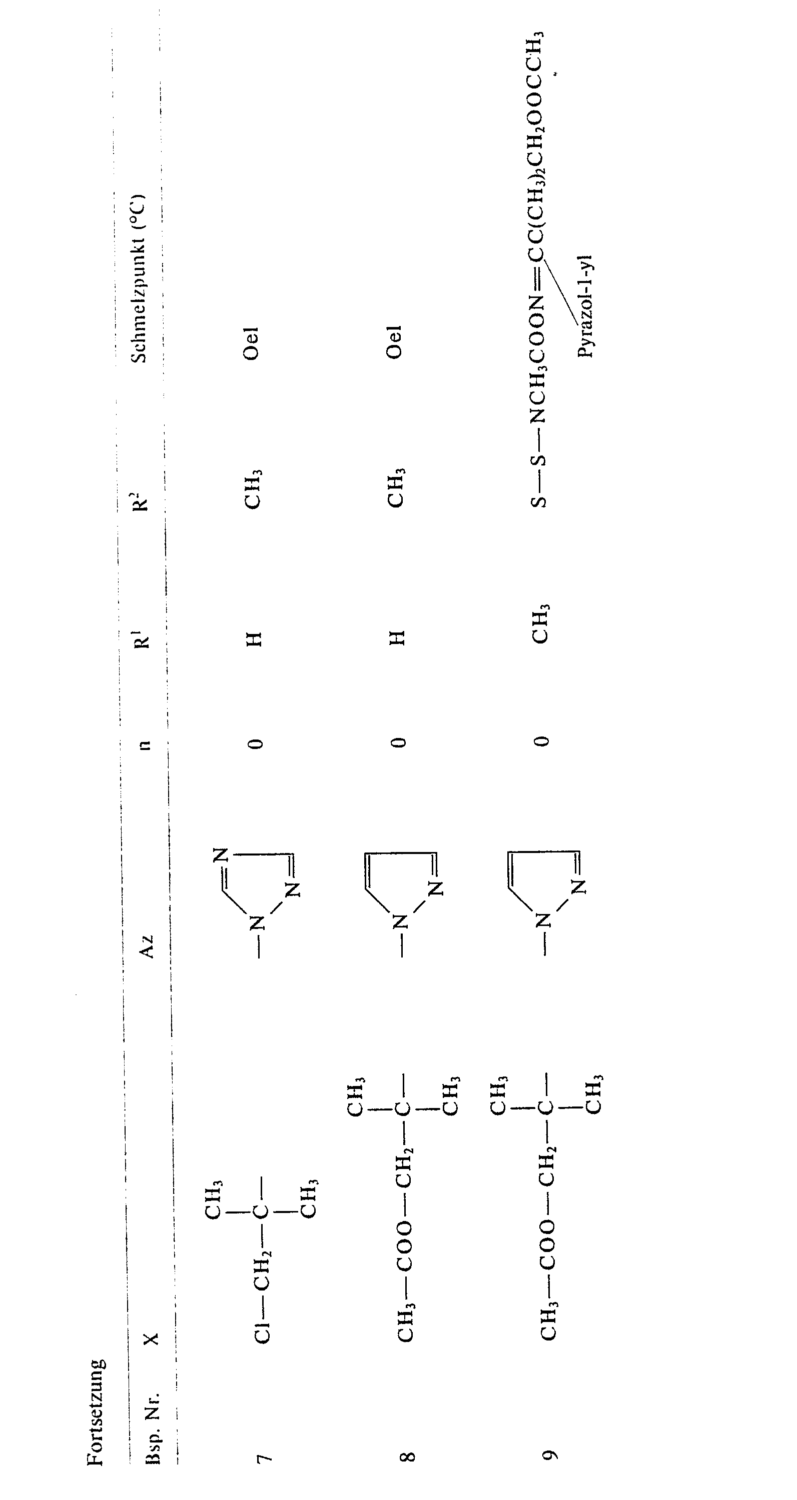

## Patentansprüche

1. Substituierte Alkanyl-azolyl-oximcarbamate der allgemeinen Formel (I) 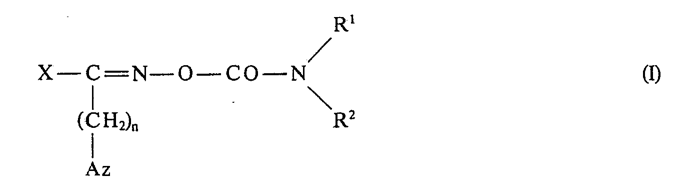 in welcher
Az für gegebenenfalls durch Chlor, Methyl, Äthyl, Nitro oder Methylmercapto substituiertes Pyrazol-1-yl, Imidazol-1-yl, 1,2,4-Triazot-l-yl, 1,2,3-Triazol-l-yl und 1,3,4-Triazol-1-yl steht,
R¹ für Wasserstoff oder Alkyl steht,
R² für Wasserstoff, Alkyl, Allyl, Halogenalkyl oder Alkoxyalkyl, sowie die Gruppe -(S)m-R³ steht, falls R¹ für Alkyl steht,
R³ für Alkyl, Halogenalkyl, gegebenenfalls durch Chlor oder Trifluormethyl substituiertes Phenyl, Alkoxycarbonyl, die Gruppe -NR⁴R⁶ sowie für den gleichen Rest steht, an den die Gruppe -(S)m-R³ gebunden ist,
R⁴ für Alkyl steht,
R⁵ für Alkyl, Dialkylcarbamoyl, Alkoxycarbonyl oder gegebenenfalls durch Halogen, Alkyl mit 1―2 Kohlenstoffatomen oder Halogenalkyl mit 1-2 Kohlenstoffatomen und bis zu 5 Halogenatomen substituiertes Phenylsulfonyl steht oder
R⁴ und R⁵ gemeinsam mit dem N-Atom für Piperidinol oder Morpholino steht,
X für Cₗ-₄-Alkyl steht, das ein- oder zweifach substituiert ist durch Halogen, Acyloxy, Carbamoyl-, Alkylcarbamoyl- und Dialkylcarbamoyloxy mit jeweils 1 bis 4 Kohlenstoffatomen in jedem Alkylteil; Alkylsulfonyloxy mit 1 bis 4 Kohlenstoffatomen, Hydroxy, Nitro, Cyano, Alkoxycarbonyl mit 1 bis 4 Kohlenstoffatomen im Alkylteil, sowie Alkoxy und Alkylthio mit jeweils 1 bis 4 Kohlenstoffatomen, wobei die Alkylgruppen von 2-Alkoxy- bzw. Alkylthio-Substituenten auch in Form eines 5- oder 6gliedrigen Ringes miteinander verbunden sein können,
m für 1 oder 2 steht und
n für 0 oder 1 steht
und deren physiologisch verträgliche Säureadditions-Salze und Metallsalz-Komplexe.

2. Alkanyl-azolyl-oximcarbamate gemäß Anspruch 1, wobei
X und Az die in Anspruch 1 angegebene Bedeutung besitzen,
R¹ für Wasserstoff und geradkettiges oder verzweigtes Alkyl mit 1 bis 4 Kohlenstoffatomen steht,
R² für Wasserstoff, geradkettiges oder verzweigtes Alkyl mit 1 bis 12 Kohlenstoffatomen, Allyl, Halogenalkyl mit bis zu 2 Kohisnstoff- und bis zu 5 Halogenatomen, sowie für Alkoxyalkyl mit bis zu 2 Kohlenstoffatomen in jedem Alkylteil steht. R² steht außerdem für die Gruppe -(S)m-R³, falls R¹ für Alkyl steht. Dabei steht R³ für geradkettiges oder verzweigtes Alkyl mit 1 bis 4 Kohlenstoffatomen, Halogenalkyl mit 1 bis 2 Kohlenstoff- und bis zu 5 Halogenatomen, gegebenenfalls chlor- oder trifluormethylsubstituiertes Phenyl sowie für Alkoxycarbonyl mit 1 bis 4 Kohlenstoffatomen im Alkoxyteil,
R³ steht ferner für den gleichen Rest, an den die Gruppe -(S)ₘR³ gebunden ist, sowie für die Gruppe -NR4R⁵;
R⁴ steht für geradkettiges oder verzweigtes Alkyl mit 1 bis 4 Kohlenstoffatomen;
R⁵ steht für geradkettiges oder verzweigtes Alkyl mit 1 bis 4 Kohlenstoffatomen in jedem Alkylteil, Alkoxycarbonyl mit 1 bis 4 Kohlenstoffatomen im Alkylteil sowie gegebenenfalls durch Halogen, Alkyl mit 1 bis 2 Kohlenstoffatomen, Halogenalkyl mit 1 bis 2 Kohlenstoff- und bis zu 5 Halogenatomen substituiertes Phenylsulfonyl;
R⁴ und R⁵ gemeinsam mit dem N-Atom, m und n besitzen die in Anspruch 1 angegebene Bedeutung.

3. Alkanyl-azolyl-oximcarbamate gemäß Anspruch 1, wobei
Az und n die in Anspruch 1 angegebene Bedeutung besitzen,
R¹ für Wasserstoff oder Methyl steht,
R² für Methyl, Methoxymethyl oder Allyl sowie die Gruppe -(S)ₘ-R³ steht,
R³ für Methoxycarbonyl, Trichlormethyl, Dichlorfluormethyl, gegebenenfalls durch Chlor oder Trifluormethyl substituiertes Phenyl und die Gruppe -NR⁴R⁵ steht, wobei m für 1 steht; oder R³ den gleichen Rest bedeutet, an den die Gruppe -(S)m-R³ gebunden ist, wobei m für 1 oder 2 steht,
R4 für Methyl steht,
R⁵ für Methyl, Methoxycarbonyl und Methylphenylsulfonyl steht,
R⁴ und R⁵ gemeinsam mit dem N-Atom für Piperidino oder Morpholino stehen,
X für gegebenenfalls einfach oder zweifach substituiertes tertiäres Butyl steht, wobei als Substituenten genannt seien: Chlor, Fluor, Brom, Hydroxy, Acetyloxy, Methylcarbamoyloxy und Dimethylcarbamoyloxy.

4. Verfahren zur Herstellung der Alkanyl-azolyl-oximcarbamate der Formel (I) nach Anspruch 1 bis 3, dadurch gekennzeichnet, daß man
a) Oxime der Formel (II) mit Phosgen und anschließend mit Aminen der Formel (V) 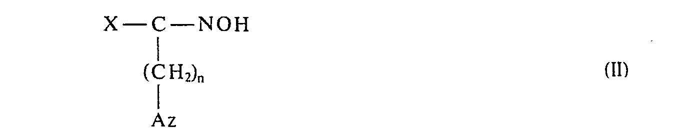 in welcher
Az, X und n die oben angegebene Bedeutung haben oder mit Carbamoylhalogeniden der Formel (III) 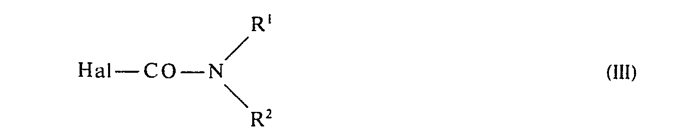 in welcher
R' und R² die oben angegebene Bedeutung haben und
Hai für Fluor oder Chlor steht,
entweder in Gegenwart eines Verdünnungsmittels und eines säurebindenden Mittels oder in Gegenwart eines Verdünnungsmittels und Natriumhydrid umsetzt, oder
b) Oxime der Formel (II) mit Isocyanaten der Formel (IV) 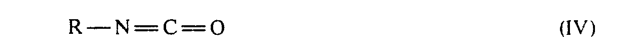 in welcher
R für Alkyl, Allyl oder Alkoxyalkyl steht,
in Gegenwart eines Verdünnungsmittels und gegebenenfalls in Gegenwart eines Katalysators umsetzt, oder
c) Oxime der Formel (II) mit Phosgen und anschließend mit Aminen der Formel (V) 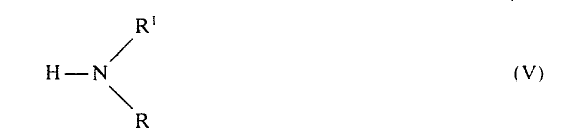 in welcher
R und R¹ die oben angegebene Bedeutung haben und
R zusätzlich für Wasserstoff steht,
entweder in Gegenwart eines Verdünnungsmittels und eines säurebindenden Mittels oder in Gegenwart eines Verdünnungsmittels und Natriumhydrid umsetzt, oder
d) gegebenenfalls die nach den Verfahren (a), (b) und (c) erhältlichen Oximcarbamate der Formel (VI) 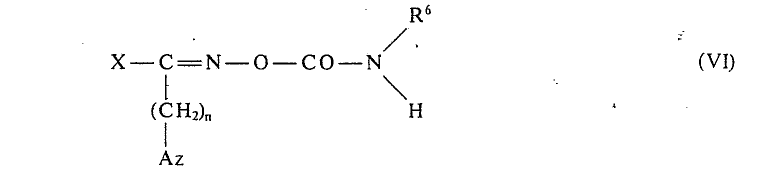 in welcher
Az, X und n die oben angegebene Bedeutung haben und
R⁶ für Alkyl mit bis 4 Kohlenstoffatomen steht,
mit Sulfenchloriden der Formel (VII) 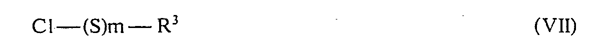 in welcher
R³ und m die oben angegebene Bedeutung haben,
in Gegenwart eines Verdünnungsmittels und eines säurebindenden Mittels umsetzt.

5. Verfahren zur Herstellung der Salze und Metallkomplexe der Alkanyl-azolyl-oximcarbamate der Formel (1), dadurch gekennzeichnet, daß man die Alkanyl-azolyl-oximcarbamate mit Säuren in ihre Salze überführt oder mit Metallsalzen in ihre Metallkomplexe überführt.

6. Insektizide, akarizide und nematizide Mittel, gekennzeichnet durch einen Gehalt an mindestens einem Alkanyloximcarbamat nach Anspruch 1 bis 3.

7. Verwendung von Alkanyl-azolyl-oximcarbamaten nach Anspruch 1 bis 3 zur Bekämpfung von Insekten, Spinntieren oder Nematoden.

8. Verfahren zur Herstellung insektizider, akarizider oder nematizider Mittel, dadurch gekennzeichnet, daß man Alkanyl-azolyl-oximcarbamate nach Anspruch 1 bis 3 mit Streckmitteln und/oder oberflächenaktiven Mitteln vermischt.

## Claims

1. Substituted alkanyl-azolyl oxime-carbamates of the general formula (I) - 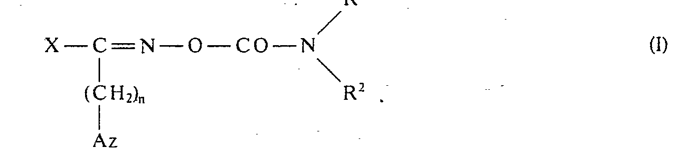
in which
Az represents pyrazol-1-yl, imidazolyl-1-yl, 1,2,4-triazol-1-yl, 1,2,3-triazol-1-yl and 1,3,4-triazol-1-yl. which are optionally substituted by chlorine, methyl, ethyl, nitro ormethylmercapto,
R¹ represents hydrogen or alkyl,
R² represents hydrogen, alkyl, allyl, halogenoalkyl or alkoxyalkyl, as well as the -(S)m-R³ group if R¹ represents alkyl,
R³ represents alkyl, halogenoalkyl, phenyl optionally substituted by chlorine or trifluoromethyl, alkoxycarbonyl, the -NR⁴R⁵ group or the same radical to which the ―(S)ₘ―R³ group is bonded,
R⁴ represents alkyl,
R⁵ represents alkyl, dialkylcarbamoyl, alkoxycarbonyl or phenylsulphonyl optionally substituted by halogen, alkyl with 1-2 carbon atoms or halogenoalkyl with 1-2 carbon atoms and up to 5 halogen atoms or
R⁴ and R⁵, together with the nitrogen atom, represent piperidinyl or morpholino,
X represents C₁-₄ alkyl, which is monosubstituted or disubstituted by halogen, acyloxy, carbamoyloxy, alkylcarbamoyloxy and dialkylcarbamoyloxy with in each case 1 to 4 carbon atoms in each alkyl part; alkylsulphonyloxy with 1 to 4 carbon atoms, hydroxy, nitro, cyano, alkoxycarbonyl with 1 to 4 carbon atoms in the alkyl part, as well as alkoxy and alkylthio with in each case 1 to 4 carbon atoms, it also being possible for the alkyl groups of 2 alkoxy or alkylthio substituents to be linked to one another in the form of a 5-membered or 6-membered ring,
m represents 1 or 2 and
n represents 0 or 1
and physiologically acceptable acid addition salts and metal salt complexes thereof.

2. Alkanyl-azolyl oxime-carbamates according to claim 1, wherein
X and Az have the meaning indicated in claim 1,
R¹ represents hydrogen and straight-chain or branched alkyl with 1 to 4 carbon atoms,
R² represents hydrogen, straight-chain or branched alkyl with 1 to 12 carbon atoms, allyl, halogenoalkyl with up to 2 carbon atoms and up to 5 halogen atoms, as well as alkoxyalkyl with up to 2 carbon atoms in each alkyl part.
R² additionally represents the -(S)m-R³ group if R¹ represents alkyl. In this group R³ represents straight-chain or branched alkyl with 1 to 4 carbon atoms, halogenoalkyl with 1 to 2 carbon atoms and up to 5 halogen atoms, phenyl optionally substituted by chlorine or trifluoromethyl, as well as alkoxycarbonyl with 1 to 4 carbon atoms in the alkoxy part,
R³ furthermore represents the same radical to which the -{S)m-R³ group ist bonded, as well as the -NR4R⁵group;
R⁴ represents straight-chain or branched alkyl with 1 to 4 carbon atoms;
R⁵ represents straight-chain or branched alkyl with 1 to 4 carbon atoms in each alkyl part, alkoxycarbonyl with 1 to 4 carbon atoms in the alkyl part as well as phenylsulphonyl optionally substituted by halogen, alkyl with 1 to 2 carbon atoms, halogenoalkyl with 1 to 2 carbon atoms and up to 5 halogen atoms;
R⁴ and R^{s} together, and m and n have the meaning indicated in claim 1.

3. Alkanyl-azolyl oxime-carbamates according to claim 1, wherein
Az, X and n have the meaning indicated in claim 1,
R¹ represents hydrogen or methyl,
R² represents methyl, methoxymethyi or allyi as well as the -(S)ₘ-R³ group,
R³ represents methoxycarbonyl, trichloromethyl, dichlorofluoromethyl, phenyl optionally substituted by chlorine or trifluoromethyl and the -NR⁴R⁵ group, m representing 1; or R³ denotes the same radical to which the -(S)m-R³ group is bonded, m representing 1 or 2,
R4 represents methyl,
R⁵ represents methyl, methoxycarbonyl and methylphenylsulphonyl,
R⁴ and R⁵ together represent piperidinyl or morpholino,
X represents tertiary butyl which is optionally monosubstituted or disubstituted, substituents which may be mentioned being: chlorine, fluorine, bromine, hydroxyl, acetyloxy, methylcarbamoyloxy and dimethylcarbamoyloxy.

4. Process for the preparation of the alkanyl-azolyl oxime-carbamates of the formula (I) according to Claim 1 to 3, characterised in that
a) oximes of the formula (II) are reacted with phosgene and the products are then reacted with amines of the formula (V) 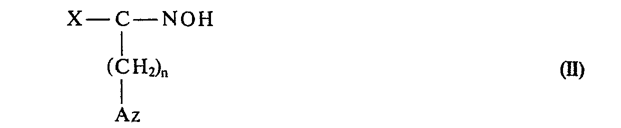 in which
Az, X and n have the meaning indicated above, with carbamoyl halides of the formula (III) 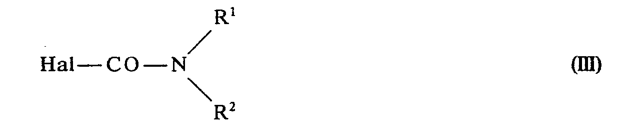 in which
R¹ and R² have the meaning indicated above and
Hal represents fluorine or chlorine,
either in the presence of a diluent and an acid-binding agent or in the presence of a diluent and sodium hydride, or
b) oximes of the formula (II) are reacted with isocyanates of the formula (IV) 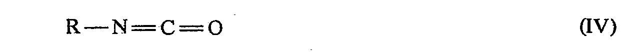 in which
R represents alkyl, allyl or alkoxyalkyl,
in the presence of a diluent and if appropriate in the presence of a catalyst, or
c) oximes of the formula (11) are reacted with phosgene and the products are then reacted with amines of the formula (V) 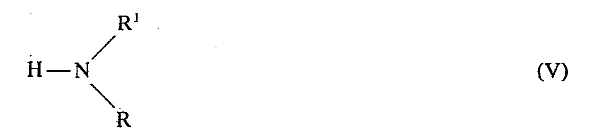 in which
R and R¹ have the meaning indicated above and
R additionally represents hydrogen,
either in the presence of a diluent and an acid-binding agent or in the presence of a diluent and sodium hydride, or
d) the oxime-carbamates obtainable by processes (a), (b) and (c), of the formula (VI) 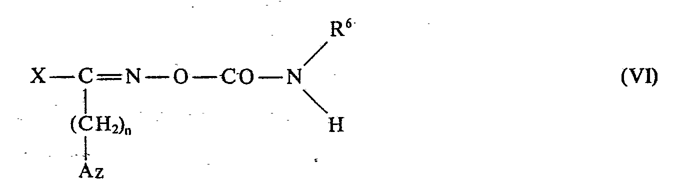 in which
Az, X and n have the meaning indicated above and
R⁶ represents alkyl with 1 to 4 carbon atoms,
are optionally reacted with sulphenyl chlorides of the formula (VII) 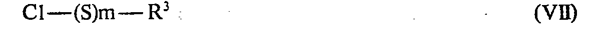 in which
R³ and m have the meaning indicated above,
in the presence of a diluent and an acid-binding agent.

5. Process for the preparation of the salts and metal complexes of the alkanyl-azolyl oxime-carbamates of the formula (I), characterised in that the alkanyl-azolyl oxime-carbamates are converted into their salts using acids or into their metal complexes using metal salts.

6. Insecticidal, acaricidal and nematicidal agents, characterised in that they contain at least one alkanyl oxime-carbamate according to Claim 1 to 3.

7. Use of alkanyl-azolyl oxime-carbamates according to Claim 1 to 3 for combating insects, arachnidae or nematodes.

8. Process for the preparation of insecticidal, acaricidal or nematicidal agents, characterised in that alkanylazolyl oxime-carbamates according to Claim 1 to 3 are mixed with extenders and/or surface-active agents.

## Revendications

1. Des carbamates d'alcanyl-azolyl-oximes substitués de formule générale (I): 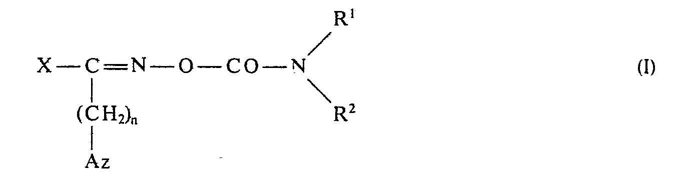 dans laquelle
Az est un groupe pyrazol-1-yle, imidazol-1-yle, 1,2,4-triazol-1-yle, 1,2,3-triazol-1-yle et 1,3,4-triazol-1- yle substitué éventuellement par du chlore, un groupe méthyle, un groupe éthyle, un groupe nitro ou un groupe méthylmercapto
R¹ est de l'hydrogène ou un groupe alkyle
R² est de l'hydrogène ou un groupe alkyle, allyle, halogénalkyle ou alkoxy-alkyle ainsi que le groupe -(S)m- R³ au cas où R¹ est un groupe alkyle
R³ est un groupe alkyle, halogénalkyle, phényle éventuellement substitué par du chlore ou un radical trifluorométhyle, alkoxycarbonyle, le groupe -NR⁴R⁵ ainsi que le même reste que celui auquel le groupe -(S)m-R3 est lié,
R⁴ est un groupe alkyle
R⁵ est un groupe alkyle, dialkylcarbamoyle, alkoxycarbonyle ou phénylsulfonyle éventuellement substitué par un halogène, un radical alkyle ayant 1 ou 2 atomes de carbone ou un radical halogénalkyle ayant 1 ou 2 atomes de carbone et jusqu'à 5 atomes d'halogènes, ou bien
R⁴ et R⁵ forment ensemble avec l'atome d'azote un groupe pipéridinyle ou morpholino
X est un groupe alkyle en Ci à C₄ qui est substitué une ou deux fois par un halogène, un groupe acyloxy, un groupe carbamoyl-, alkylcarbamoyl- ou dialkylcarbamoyloxy ayant chacun 1 à 4 atomes de carbone dans chaque partie alkyle; un groupe alkylsulfonyloxy ayant 1 à 4 atomes de carbone, un groupe hydroxy, nitro, cyano, alkoxycarbonyle ayant 1 à 4 atomes de carbone dans la partie alkyle, de même qu'un groupe alkoxy ou alkylthio ayant chacun 1 à 4 atomes de carbone, les groupes alkyle des substituants 2-alkoxy et alkylthio pouvant également être liés ensemble sous la forme d'un noyau pentagonal ou hexagonal
m est égal à 1 ou 2, et
n est égal à 0 ou 1
et leurs sels d'addition d'acides acceptables du point de vue physiologique et leurs complexes de sels métalliques.

2. Des carbamates d'alcanyl-azolyl-oximes suivant la revendication 1, dans lesquels:
X et Az ont la définition indiquée dans la revendication 1
R¹ est de l'hydrogène et un groupe alkyle à chaîne droite ou ramifiée ayant 1 à 4 atomes de carbone,
R² est de l'hydrogène, un groupe alkyle à chaîne droite ou ramifiée ayant 1 à 12 atomes de carbone, un groupe allyle, halogénalkyle ayant jusqu'à 2 atomes de carbone et jusqu'à 5 atomes d'halogènes ainsi qu'un groupe alkoxy-alkyle ayant jusqu'à 2 atomes de carbone dans chaque radical alkyle, R² représente en outre le groupe -(S)m-R³ au cas où R' est un groupe alkyle, R³ désignant un groupe alkyle à chaîne droite ou ramifiée ayant 1 à 4 atomes de carbone, un groupe halogénalkyle ayant 1 ou 2 atomes de carbone et jusqu'à 5 atomes d'halogènes, un groupe phényle éventuellement substitué par du chlore ou par un groupe trifluorométhyle ainsi qu'un groupe alkoxycarbonyle ayant 1 à 4 atomes de carbone dans la partie alkoxy,
R³ désigne en outre le même reste que celui auquel le groupe -(S)m-R³ est lié, ainsi que le groupe -NR4R₅,
R⁴ est un groupe alkyle à chaîne droite ou ramifiée ayant 1 à 4 atomes de carbone,
R⁵ est un groupe alkyle à chaîne droite ou ramifiée ayant 1 à 4 atomes de carbone dans chaque partie alkyle, un groupe alkoxycarbonyle ayant 1 à 4 atomes de carbone dans la partie alkyle ainsi qu'un groupe phénylsulfonyle éventuellement substitué par un halogène, un radical alkyle ayant 1 ou 2 atomes de carbone, un radical halogénalkyle ayant 1 ou 2 atomes de carbone et jusqu'à 5 atomes d'halogènes,
R⁴ et R⁵ considérés ensemble, m et n ayant les définitions indiquées dans la revendication 1.

3. Des carbamates d'alcanyl-azolyl-oximes suivant la revendication 1, dans lesquels
Az, X et n ont les définitions indiquées dans la revendication 1,
R¹ est de l'hydrogène ou un groupe méthyle,
R² est un groupe méthyle, méthoxyméthyle ou allyle ainsi que le groupe ―(S)ₘ―R³
R³ est un groupe méthoxycarbonyle, trichlorométhyle, dichlorofluorométhyle, phényle éventuellement substitué par du chlore ou par un radical trifluorométhyle et le groupe ―NR⁴R⁵, m étant égal à 1, ou bien R³ désigne le même reste que celui auquel le groupe ―(S)ₘ―R³ est lié, m étant égal à 1 ou 2,
R⁴ est un groupe méthyle,
R⁵ est un groupe méthyle, méthoxycarbonyle ou méthylphénylsulfonyle
R⁴ et Reforment ensemble le groupe pipéridinyle ou morpholino
X est un groupe butyle tertiaire éventuellement substitué une ou deux fois, les substituants pouvant être du chlore, du fluor, du brome ou un groupe hydroxy, acétyloxy, méthylcarbamoyloxy ou diméthylcarbamoyloxy.

4. Procédé de production des carbamates d'alcanyl-azolyl-oximes de formule (I) suivant les revendications 1 à 3, caractérisé en ce que
(a) on fait réagir des oximes de formule (II) avec du phosgène, puis avec des amines de formule (V) 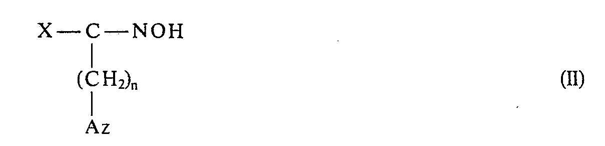 dans laquelle Az, X et n ont les définitions indiquées ci-dessus,
avec des halogénures de carbamoyle de formule (III): 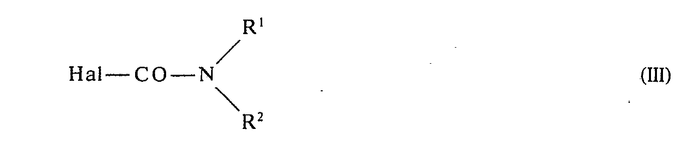 dans laquelle
R¹ et R² ont les définitions données ci-dessus et Hal est du fluor ou du chlore,
en présence d'un diluant et d'un accepteur d'acide ou en présence d'un diluant et d'hydrure de sodium, ou bien
(b) on fait réagir des oximes de formule (II) avec des isocyanates de formule (IV) 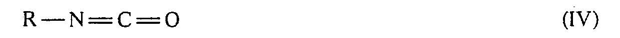 dans laquelle
R est un groupe alkyle, allyle ou alkoxy-alkyle,
en présence d'un diluant et, le cas échéant, en présence d'un catalyseur, ou
(c) on fait réagir des oximes de formule (II) avec du phosgène, puis avec des amines de formule (V) 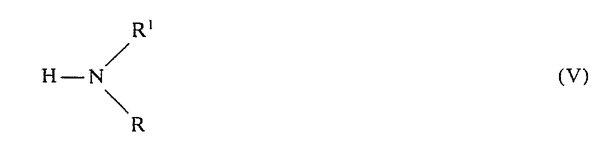 dans laquelle
R et R¹ ont les définitions indiquées ci-dessus et R représente, en outre, de l'hydrogène,
en présence d'un diluant et d'un accepteur d'acide ou en présence d'un diluant et d'hydrure de sodium, ou
(d) on fait réagir éventuellement les carbamates d'oximes obtenus par les procédés (a), (b) et (c), de formule (VI): 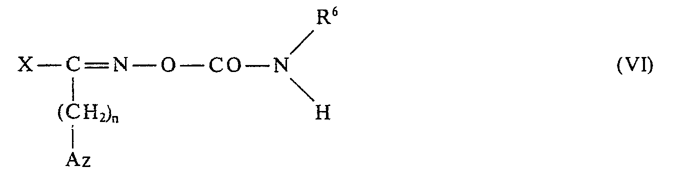 dans laquelle
Az, X et n ont les définitions indiquées ci-dessus et
R⁶ est un groupe alkyle ayant 1 à 4 atomes de carbone,
avec des chlorures de sulfényle de formule (VII) 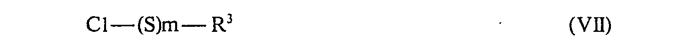 dans laquelle
R³ et m ont les définitions indiquées ci-dessus,
en présence d'un diluant et d'un accepteur d'acide.

5. Procédé de production des sels et complexes métalliques des carbamates d'alcanyl-azolyl-oximes de formule (1), caractérisé en ce qu'on transforme les carbamates d'alcanyl-azolyl-oximes avec des acides en leurs sels, ou on les transforme avec des sels métalliques en leurs complexes métalliques.

6. Compositions insecticides, acaricides et nématicides, caractérisées par une teneur en au moins un carbamate d'alcanyl-oxime suivant les revendications 1 à 3.

7. Utilisation de carbamates d'alcanyl-azolyl-oximes suivant les revendications 1 à 3 pour la lutte contre des insectes, des acariens ou des nématodes.

8. Procédé de production de compositions insecticides, acaricides ou nématicides, caractérisé en ce qu'on mélange des carbamates d'alcanyl-azolyl-oximes suivant les revendications 1 à 3 avec des diluants et/ou des agents tensio-actifs.
